# EUROPÄISCHE PATENTANMELDUNG

(11) **EP 3 399 016 A2**
(43) Veröffentlichungstag der Anmeldung: **07.11.2018**
(21) Anmeldenummer: 18165606.7
(22) Anmeldetag: 04.04.2018
(51) Int. Cl.: C12M 1/107, C12M 1/00

(54) **VERFAHREN ZUM BETRIEB EINER BIOGASANLAGE UND ZUR NUTZUNG VON GÜLLE**

(30) Priorität: 11.04.2017 DE 102017206190
(71) Anmelder: Röhren- und Pumpenwerk Bauer Ges.mbH, 8570 Voitsberg (AT)
(72) Erfinder: EICHLER, Dietrich, 01824 Königstein (DE); ROISS, Otto, 8010 Graz (AT)
(74) Vertreter: Hoefer & Partner Patentanwälte mbB

(57) **Zusammenfassung**

Die Erfindung betrifft ein Verfahren zum Betrieb einer Biogasanlage (1) umfassend die folgenden Schritte: Separieren von Gülle (17) in Feststoff (23) und Flüssigkeit (18), bevor die Gülle (17) einem Biogasreaktor (2) zugeführt wird, Zuführen der separierten Flüssigkeit (18) in einen Biogasreaktor (2), und Verwenden des separierten Feststoffs (23) als Holzersatzstoff zur Herstellung von Faserformteilen.

## Beschreibung

Die Erfindung betrifft ein Verfahren zum Betrieb einer Biogasanlage, ein Verfahren zur Nutzung von Gülle sowie entsprechende Anlagen zur Durchführung der Verfahren.

Der Stand der Technik kennt unterschiedliche Biogasanlagen. Diese weisen üblicherweise zumindest einen Biogasreaktor auf, in dem Gülle und/oder pflanzliches Substrat vergast werden. Das entstehende Gas kann auf unterschiedliche Weise genutzt werden, beispielsweise in einem Blockheizkraftwerk.

Des Weiteren kennt der Stand der Technik verschiedene Herstellungsmethoden für Faserformteile. Dabei werden insbesondere Fasern aus Holz zusammen mit einem Bindemittel in Formteile, beispielsweise Platten gepresst. Diese Formteile werden beispielsweise als MDF-Platten bezeichnet. Die Fasern aus Holz können dabei vollständig oder teilweise durch andere pflanzliche Fasern ersetzt werden, die aus Gärresten der Biogasproduktion gewonnen werden.

Es ist Aufgabe der Erfindung ein Verfahren zum Betrieb einer Biogasanlage anzugeben, das eine möglichst effiziente Ausnutzung der Rohstoffe ermöglicht. Weitere Aufgaben können sich aus den hier beschriebenen Verfahren und Anlagen sowie deren Vorteile ergeben.

Die Lösung der Aufgabe erfolgt durch die Merkmale der unabhängigen Ansprüche. Die abhängigen Ansprüche haben vorteilhafte Ausgestaltungen der Erfindung zum Gegenstand.

### Erster Aspekt:

Die Erfindung offenbart ein Verfahren zum Betrieb einer Biogasanlage. Bei dem Verfahren erfolgt ein Separieren von Gülle in Feststoff und Flüssigkeit. Vorzugsweise wird Gülle von nicht-wiederkäuenden Säugetieren, beispielsweise Schweinen, verwendet. Allerdings kann auch andere Gülle, beispielsweise von Rindern oder Geflügel, verwendet werden.

Der separierte Feststoff ist wesentlich trockener als die separierte Flüssigkeit. Das Separieren erfolgt, bevor die Gülle einem Biogasreaktor zugeführt wird. So handelt es sich bei der zu separierenden Gülle zumindest teilweise um Gülle, die noch nicht zur Gasproduktion genutzt wurde. Der Ausdruck "Gülle" steht für tierische Exkremente, gegebenenfalls mit pflanzlichen Zusatzstoffen, wie beispielsweise Einstreu. Das Separieren der Gülle erfolgt vorzugsweise in einem Pressschneckenseparator.

Die separierte Flüssigkeit wird dem Biogasreaktor zugeführt und im Biogasreaktor zur Produktion von Biogas verwendet. Bei dem Biogasreaktor handelt es sich insbesondere um zumindest einen Fermenter und/oder zumindest einen Nachgärer. Vorzugsweise wird zusätzlich zu der separierten Flüssigkeit pflanzliches Substrat dem Biogasreaktor zugeführt. Insbesondere wird zumindest ein Teil der separierten Flüssigkeit außerhalb des Biogasreaktors mit dem pflanzlichen Substrat vermischt und die Mischung dem Biogasreaktor zugeführt.

Der separierte Feststoff wird erfindungsgemäß zur Herstellung von Faserformteilen verwendet. Insbesondere eigenen sich hierzu die lignocellulosehaltigen Fasern im Feststoff. Somit fungiert der aus Gülle separierte Feststoff als Holzersatzstoff. Bei den Faserformteilen handelt es sich insbesondere um Faserplatten, beispielsweise MDF- Platten. Allerdings können auch nicht-plattenförmige Faserformteile aus dem separierten Feststoff hergestellt werden. Der Feststoff kann zur Herstellung der Faserformteile auch mit weiteren Fasern, beispielsweise Holzfasern, vermischt werden. Darüber hinaus zählen auch Brennstoffpellets zu den Faserformteilen, die vollständig oder teilweise aus dem separierten Feststoff hergestellt werden können.

Im Rahmen der Erfindung wurde erkannt, dass die relativ großen Fasern im separierten Feststoff für die Herstellung der Faserformteile wesentlich besser geeignet sind, wenn sie nicht den Biogasreaktor durchlaufen und durch die Gärprozesse geschädigt werden. Bei dem Separieren der Gülle verbleiben leicht verwertbare Inhaltsstoffe und die kurzen Faseranteile in der separierten Flüssigkeit und stehen im Biogasreaktor zur Erzeugung des Biogases zur Verfügung. Es wurde erkannt, dass der separierte Feststoff mit den relativ langen Fasern für den Gärprozess eine untergeordnete Rolle spielt, da diese Fasern im Biogasreaktor schwer zu verwerten sind. Durch die Stickstoffhemmung im Biogasreaktor verringert sich der anaerobe Prozess, sodass oftmals mehr als 50 % der Trockenmasse, vorrangig die großen Fasern, im Gärrest übrig bleiben. Deshalb werden erfindungsgemäß diese großen Fasern noch vor dem Gärprozess der Gülle entnommen und können unter Umgehung der Gärung für die Produktion der Faserformteile verwendet werden.

Eine gegebenenfalls benötigte Menge an lignocellulosehaltigem Material im Biogasreaktor kann ohne weiteres durch das Beimischen des pflanzlichen Substrats erreicht werden.

Des Weiteren ist bevorzugt vorgesehen, den Gärrest zumindest teilweise der zu separierenden Gülle beizumischen. Dieser Anteil des Gärrests durchläuft dadurch zusammen mit der frischen Gülle den Prozessschritt des Separierens.

Insbesondere ist vorgesehen, den Gärrest zumindest teilweise einem Feinfilter zuzuführen. In dem Feinfilter werden insbesondere kleinere Fasern herausgefiltert als beim Separieren. Das im Feinfilter erzeugte Filterkonzentrat wird, wie oben beschrieben, der zu separierenden Gülle beigemischt. Der Klarlauf aus dem Feinfilter wird vorzugsweise einem Endlager der Biogasanlage zugeführt, kann vor dem Endlager noch einen Nachgärer durchlaufen oder kann anderweitig genutzt werden.

Bei dem Feinfilter handelt es sich insbesondere um einen Druckfilter bei dem der Gärrest durch einen Vibrationsantrieb in Vibration versetzt wird und dadurch durch ein entsprechendes Sieb gedrückt bzw. bewegt wird. Die Patentschrift DE 100 47 431 B4 zeigt einen Druckfilter mit Vibrationsantrieb, der für das beschriebene Verfahren vorzugsweise als Feinfilter verwendet werden kann.

Vorzugsweise wird ein Güllesammelbehälter genutzt, aus dem die Gülle über eine Zuleitung in einen Zulauf eines Separators gefördert wird. Vorzugsweise ist der Auslauf des Feinfilters für das Filterkonzentrat sowohl an den Güllebehälter als auch an die Zuleitung oder den Zulauf angeschlossen. Dadurch kann wahlweise das Filterkonzentrat der Gülle im Güllesammelbehälter beigemischt werden oder es erfolgt ein direktes Einleiten in die dem Separator zufließende Gülle. Dadurch ist es möglich, den Feuchtigkeitsgehalt der zu separierenden Gülle zu regulieren.

Vorzugsweise wird im Feinfilter eine Feinfilter-Maschenweite mit folgender Spezifikation genutzt: Bei der Maschenweite handelt es sich insbesondere um einen Lochdurchmesser eines Gewebesiebes, welches über einen Stützkorb stabilisiert ist, oder die Spaltweite eines Stabsiebes. Vorzugsweise wird in dem Feinfilter, insbesondere ausgebildet als Druckfilter mit Vibrationsantrieb, das Stabsieb verwendet. Bevorzugt liegt die Obergrenze der verwendeten Maschenweite bei höchstens 0,3 mm, insbesondere höchstens 0,2 mm, besonders vorzugsweise höchstens 0,1 mm. Bevorzugt liegt die Untergrenze der verwendeten Maschenweite bei mindestens 0,01 mm, vorzugsweise mindestens 0,05 mm, besonders vorzugsweise mindestens 0,07 mm.

Vorzugsweise wird zum Separieren, insbesondere im Pressschneckenseparator, eine Separier-Spaltweite mit folgender Spezifikation genutzt: Vorzugsweise wird zum Separieren ein Stabsieb verwendet. Die Spaltweite zum Separieren ist vorzugsweise größer als die Spaltweite bzw. die Maschenweite im Feinfilter. Bevorzugt liegt die Obergrenze der verwendeten Separier-Spaltweite, bei höchstens 1 mm, insbesondere höchstens 0,8 mm, besonders vorzugsweise höchstens 0,6 mm. Bevorzugt liegt die Untergrenze bei mindestens 0,1 mm, vorzugsweise mindestens 0,3 mm, besonders vorzugsweise mindestens 0,4 mm.

Wie bereits beschrieben, wird der separierte Feststoff zumindest teilweise zum Herstellen der Faserformteile verwendet. Dabei ist insbesondere vorgesehen, dass der separierte Feststoff vor dem Herstellen der Faserformteile aufbereitet wird. Bei diesem Aufbereiten wird der Feststoff insbesondere getrocknet und/oder gereinigt und/oder sortiert.

Das Verfahren umfasst vorzugsweise den Schritt der Herstellung der Faserformteile. Dabei wird der separierte Feststoff, insbesondere in aufbereiteter Form, zusammen mit einem Bindemittel zu dem Faserformteil verpresst. Der aus Gülle gewonnene Feststoff kann dabei mit anderen Fasern vermischt werden.

### Zweiter Aspekt:

Gemäß einem zweiten Aspekt umfasst die vorliegende Erfindungen ein Verfahren zum Betrieb einer Biogasanlage mit den folgenden Schritten: Zunächst erfolgt ein Zuführen der Gülle und/oder von pflanzlichem Substrat in den Biogasreaktor. Der Gärrest aus dem Biogasreaktor wird zumindest teilweise dem bereits beschriebenen Feinfilter zugeführt. Das im Feinfilter erzeugte Filterkonzentrat wird zumindest teilweise als Substrat wieder in denselben Biogasreaktor geführt. So können der Feinfilter nach dem Biogasreaktor und die Rückführung des Filterkonzentrats auch unabhängig von der Verwendung der großen Fasern als Holzersatzstoff zum Einsatz kommen.

Vorzugsweise wird die Gülle vor dem Zuführen in den Biogasreaktor, wie vorab beschrieben, separiert. Das Filterkonzentrat wird vorzugsweise zumindest teilweise der zu separierenden Gülle beigemischt. Die separierte Flüssigkeit wird dem Biogasreaktor zugeführt. Der separierte Feststoff kann teilweise auch dem Biogasreaktor zugeführt werden oder anderweitig genutzt werden. Vorzugsweise wird zum Separieren die bereits beschriebene Separier-Spaltweite genutzt.

Wie bereits beschrieben, handelt es sich bei dem Feinfilter insbesondere um einen Druckfilter bei dem der Gärrest durch einen Vibrationsantrieb in Vibration versetzt wird und dadurch durch ein entsprechendes Sieb gedrückt bzw. bewegt wird. Vorzugsweise wird im Feinfilter die bereits beschriebene Feinfilter-Spalt- bzw. Maschenweite genutzt.

Vorzugsweise wird ein Güllesammelbehälter genutzt, aus dem die Gülle über eine Zuleitung in einen Zulauf eines Separators gefördert wird. Vorzugsweise ist der Auslauf des Feinfilters für das Filterkonzentrat sowohl an den Güllebehälter als auch an die Zuleitung oder den Zulauf angeschlossen. Dadurch kann wahlweise das Filterkonzentrat der Gülle im Güllesammelbehälter beigemischt werden oder es erfolgt ein direktes Einleiten in die dem Separator zufließende Gülle.

Vorzugsweise umfasst die Erfindung eine Biogasanlage, ausgebildet zur Durchführung eines der Verfahren nach dem ersten Aspekt oder den zweiten Aspekt. Die Biogasanlage umfasst vorzugsweise einen Pressschneckenseparator zum Separieren der Gülle und den beschriebenen Feinfilter. Des Weiteren umfasst die Biogasanlage vorzugsweise zumindest einen Biogasreaktor und die entsprechenden Vorrichtungen zum Bewegen der Medien.

### Dritter Aspekt:

Gemäß einem dritten Aspekt umfasst die Erfindung ein Verfahren zur Nutzung von Gülle mit den folgenden Schritten: zunächst erfolgt ein Separieren der Gülle in Feststoff und Flüssigkeit, ohne dass die Gülle zuvor einen Biogasreaktor zugeführt wird. Dadurch sind die in der Gülle enthaltenen, relativ großen Fasern nicht durch den Gärprozess beschädigt. Wie im Rahmen des ersten Aspekts vorliegender Erfindung beschrieben, wird der separierte Feststoff zumindest teilweise als Holzersatzstoff zur Herstellung von Faserformteilen verwendet.

Im Rahmen des dritten Aspekts vorliegender Erfindung bleibt offen, wie die separierte Flüssigkeit genutzt wird. Dies kann, wie beschrieben in einem Biogasreaktor erfolgen. Alternativ kann die separierte Flüssigkeit auch anderweitig genutzt werden; beispielsweise direkt auf ein Feld aufgebracht werden.

Das Separieren der Gülle erfolgt vorzugsweise mit der beschriebenen Separier-Spaltweite und in einem Pressschneckenseparator.

Die im Rahmen des ersten Aspekts beschriebenen Merkmale zur Herstellung der Faserformteile und/oder zur Aufbereitung der Fasern aus dem separierte Feststoff finden entsprechend vorteilhafte Anwendungen im Rahmen des dritten Aspekts.

Weitere Einzelheiten, Vorteile und Merkmale der vorliegenden Erfindung ergeben sich aus nachfolgender Beschreibung eines Ausführungsbeispiels anhand der Zeichnung. Es zeigt:

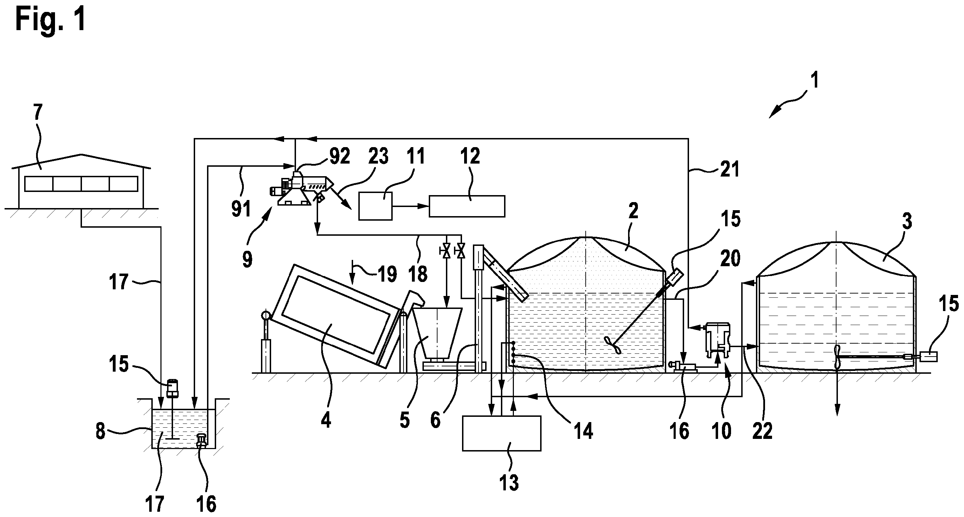

- Fig. 1: eine erfindungsgemäße Biogasanlage zur Durchführung des erfindungsgemäßen Verfahrens.

Figur 1 zeigt eine Biogasanlage 1 und eine Stallanlage 7. Aus der Stallanlage 7 fließt Gülle 17 in einen Güllesammelbehälter 8 der Biogasanlage 1.

Die Biogasanlage 1 umfasst einen Biogasreaktor 2, insbesondere ausgebildet als Fermenter, und ein nachgeschaltetes Endlager 3. Ferner umfasst die Biogasanlage 1 ein Feststoffsilo 4 für pflanzliches Substrat 19. Über einen Mischer 5 und einen Förderer 6 kann das pflanzliche Substrat 19 in den Biogasreaktor 2 gefördert werden.

Das im Biogasreaktor 2 und dem Endlager 3 gewonnene Biogas wird im gezeigten Beispiel in einem Blockheizkraftwerk 13 genutzt. Über eine Heizung 14 wird die Abwärme des Blockheizkraftwerks 13 zum Erwärmen der Biomasse im Biogasreaktor 2 verwendet.

Die Biogasanlage 1 umfasst einen Separator 9, insbesondere ausgebildet als Pressschneckenseparator. Aus dem Güllesammelbehälter 8 wird die Gülle 17 über eine Zuleitung 91 in einen Zulauf 92 des Separators 9 gefördert. Im Separator 9 wird die Gülle 17 in eine separierte Flüssigkeit 18 und in Feststoff 23 separiert.

Die separierte Flüssigkeit 18 kann wahlweise direkt dem Biogasreaktor 2 oder dem Mischer 5 zugeführt werden.

Der Gärrest 20 aus dem Biogasreaktor 2 wird einem Feinfilter 10 zugeführt. Der Klarlauf 22 aus dem Feinfilter 10 wird dem Endlager 3 zugeführt. Das Filterkonzentrat 21 aus dem Feinfilter 10 kann wahlweise in den Güllesammelbehälter 8 oder direkt in die Zuleitung 91 bzw. den Zulauf 92 geführt werden.

In dem Separator 9 wird insbesondere die bereits beschriebene Spaltweite verwendet. Der Feinfilter 10 ist, wie bereits beschrieben, vorzugsweise als Druckfilter mit Vibrationsantrieb ausgebildet. In dem Feinfilter 10 wird vorzugsweise die beschriebene Spalt- bzw. Maschenweite verwendet.

Zum Fördern der Medien umfasst die Biogasanlage 1 Pumpen 16; im gezeigten Beispiel im Güllesammelbehälter 8 und zwischen dem Biogasreaktor 2 und dem Feinfilter 10. Des Weiteren sind mehrere Rührwerke 15 vorgesehen; beispielsweise im Güllesammelbehälter 8, im Biogasreaktor 2 und dem Endlager 3.

Der Feststoff 23 aus dem Separator 9 wird in einer Aufbereitungsanlage 11 gereinigt und/oder getrocknet und/oder sortiert. Dadurch werden aus dem Feststoff 23 Fasern gewonnen, die in einer Faserformteilanlage 12, gegebenenfalls unter Beimischung weiterer Fasern, zusammen mit einem Bindemittel zu Faserformteilen gepresst werden.

Mit dem gezeigten Separator 9, der Aufbereitungsanlage 11 und der Faserformteileanlage 12 können auch unabhängig von der Erzeugung von Biogas entsprechende Fasern aus der Gülle separiert und zur Herstellung von Faserformteilen verwendet werden.

Des Weiteren ist es mit der gezeigten Anlage auch möglich, unabhängig von der Verwendung des Feststoffs 23, den Gärrest 20 im Feinfilter 10 zu filtern und das Filterkonzentrat 21, insbesondere über den Separator 9, wieder dem Biogasreaktor 2 zuzuführen.

Neben der vorstehenden schriftlichen Beschreibung der Erfindung wird zu deren ergänzender Offenbarung hiermit explizit auf die zeichnerische Darstellung der Erfindung in Figur 1 Bezug genommen.

### Bezugszeichenliste

- 1: Biogasanlage
- 2: Biogasreaktor, insbesondere Fermenter
- 3: Endlager
- 4: Feststoffsilo
- 5: Mischer
- 6: Förderer
- 7: Stallanlage
- 8: Güllesammelbehälter
- 9: Separator, insbesondere Pressschneckenseparator
- 91: Zuleitung
- 92: Zulauf
- 10: Feinfilter
- 11: Aufbereitungsanlage
- 12: Faserformteilanlage
- 13: Blockheizkraftwerk
- 14: Heizung
- 15: Rührwerk
- 16: Pumpe
- 17: Gülle
- 18: separierte Flüssigkeit
- 19: pflanzliches Substrat
- 20: Gärrest
- 21: Filterkonzentrat
- 22: Klarlauf
- 23: Feststoff mit Fasern

## Patentansprüche

1. Verfahren zum Betrieb einer Biogasanlage (1) umfassend die folgenden Schritte:
• Separieren von Gülle (17) in Feststoff (23) und Flüssigkeit (18), bevor die Gülle (17) einem Biogasreaktor (2) zugeführt wird,
• Zuführen der separierten Flüssigkeit (18) in einen Biogasreaktor (2), und
• Verwenden des separierten Feststoffs (23) als Holzersatzstoff zur Herstellung von Faserformteilen.

2. Verfahren nach Anspruch 1, wobei ein Gärrest (20) aus dem Biogasreaktor (2) zumindest teilweise der zu separierenden Gülle (17) beigemischt wird.

3. Verfahren nach Anspruch 2, wobei der Gärrest (20) aus dem Biogasreaktor (2) einem Feinfilter (10) zugeführt wird, wobei das im Feinfilter (10) erzeugte Filterkonzentrat (21) der zu separierenden Gülle (17) zugeführt wird.

4. Verfahren nach Anspruch 3, wobei ein Güllesammelbehälter (8) genutzt wird, aus dem die Gülle (17) über eine Zuleitung in einen Zulauf eines Separator (9) gefördert wird, wobei das Filterkonzentrat (21) wahlweise dem Güllesammelbehälter (8) oder direkt in die Zuleitung (91) bzw. den Zulauf (92) zuführbar ist.

5. Verfahren nach einem der Ansprüche 3 oder 4, wobei in dem Feinfilter (10) eine Feinfilter-Spalt- bzw. Maschenweite, insbesondere als Spaltweite eines Stabsiebes, genutzt wird, von
• höchstens 0,3 mm, vorzugsweise höchstens 0,2 mm, besonders vorzugsweise höchstens 0,1 mm und/oder
• mindestens 0,01 mm, vorzugsweise mindestens 0,05 mm, besonders vorzugsweise mindestens 0,07 mm.

6. Verfahren nach einem der vorhergehenden Ansprüche, wobei zum Separieren eine Spaltweite eines Stabsiebes genutzt wird, von
• höchstens 1 mm, vorzugsweise höchstens 0,8 mm, besonders vorzugsweise höchstens 0,6 mm und/oder
• mindestens 0,1, vorzugsweise mindestens 0,3 mm, besonders vorzugsweise mindestens 0,4 mm.

7. Verfahren nach einem der vorhergehenden Ansprüche, wobei der separierte Feststoff (23) vor dem Herstellen der Faserformteile, vorzugsweise durch Trocknen und/oder Reinigen und/oder Sortieren, aufbereitet wird.

8. Verfahren nach einem der vorhergehenden Ansprüche, umfassend den Schritt der Herstellung der Faserformteile, wobei der separierte Feststoff (23) zusammen mit einem Bindemittel zu dem Faserformteil verpresst wird.

9. Verfahren zum Betrieb einer Biogasanlage (1) umfassend die folgenden Schritte:
• Zuführen von Gülle (17) und/oder pflanzlichem Substrat (19) in einen Biogasreaktor (2),
• Zuführen eines Gärrests (20) aus dem Biogasreaktor (2) in einen Feinfilter (10),
• Filtern des Gärrests (20) im Feinfilter (10), und
• Zuführen des im Feinfilter (10) erzeugten Filterkonzentrats (21) als Substrat in den Biogasreaktor (2).

10. Biogasanlage (1) ausgebildet zur Durchführung eines der Verfahren nach einem der vorhergehenden Ansprüche.

11. Verfahren zur Nutzung von Gülle (17) umfassend die folgenden Schritte:
• Separieren der Gülle (17) in Feststoff (23) und Flüssigkeit (18), ohne dass die Gülle (17) zuvor einem Biogasreaktor (2) zugeführt wird, und
• Verwenden des separierten Feststoffs (23) als Holzersatzstoff zur Herstellung von Faserformteilen.
